# NEUE EUROPÄISCHE PATENTSCHRIFT

(11) **EP 2 991 615 B2**
(45) Veröffentlichungstag und Bekanntmachung des Hinweises auf die Entscheidung über den Einspruch: **15.12.2021**
(45) Hinweis auf die Patenterteilung: 04.07.2018
(21) Anmeldenummer: 14702271.9
(22) Anmeldetag: 03.02.2014
(51) Int. Cl.: A61K 8/49, A61Q 5/02, A61Q 5/12, A61K 8/73, A61K 8/46, A61K 8/44, A61K 8/37, A61K 8/92

(54) **PFLEGESHAMPOO**
HAIR CARE SHAMPOO
SHAMPOING TRAITANT

(30) Priorität: 02.05.2013 DE 102013208056
(43) Veröffentlichungstag der Anmeldung: 09.03.2016
(73) Patentinhaber: Henkel AG & Co. KGaA, 40589 Düsseldorf (DE)
(72) Erfinder: SCHRÖDER, Thomas, 22395 Hamburg (DE); HENTRICH, Dirk, 22457 Hamburg (DE); WASSEN, Marine, L-1839 Luxemburg (LU)
(74) Vertreter: LKGLOBAL Lorenz & Kopf PartG mbB Patentanwälte
(86) Internationale Anmeldenummer: PCT/EP2014/052048
(87) Internationale Veröffentlichungsnummer: WO 2014/177291

(56) Entgegenhaltungen:
- EP-A1- 2 335 681
- EP-A1- 2 415 454
- EP-A2- 1 043 010
- WO-A1-2004/078159
- WO-A2-2010/108738
- WO-A2-2012/110386
- WO-A2-2013/098066
- DE-A1-102011 078 952
- US-A- 6 056 948
- Anonymous: "Guide to Essential Oils", www.cranberrylane.com website, 24. Oktober 2003 (2003-10-24), Seiten 1-2, XP055114855, Internet Gefunden im Internet: URL:http://www.cranberrylane.com/downloads /guide-to-essential-oils-bws.pdf [gefunden am 2014-04-23]
- "Die kosmetischen Präparate", Andreas Domsch, 1992, page 212,
- "Ausdruck zu Aprikosenkernöl", , Retrieved from the Internet: URL:https://www.aprikosenkernoel.info/
- "PEG-80 Sorbitan Laurate, Band 2" In: "International Cosmetic Ingredient Dictionary and Handbook (thirteenth edition)", 2010

## Beschreibung

Die Erfindung betrifft eine reinigende kosmetische Zusammensetzung, die in einem kosmetischen Träger neben anionischem Tensid, Sorbitansesquioctanoat, kationisches Polysaccharid und gegebenenfalls amphoteres Tensid enthält. Eine solche Zusammensetzung eignet sich beispielsweise als Haarshampoo zur Reinigung der Haare.

Kosmetische Reinigungsmittel, wie beispielsweise Haarshampoos, basieren auf klassischen anionischen, amphoteren, zwitterionischen, nichtionischen und/oder kationischen Tensiden, wobei aufgrund ihres hervorragenden Reinigungs- und Schaumvermögens typischerweise überwiegend anionische Tenside, gegebenenfalls in der Mischung mit geringen Mengen an Co-Tensiden, eingesetzt werden. Entsprechende handelsübliche Shampoos reinigen die Haare und beseitigt Talg- und/oder Rückstände von Stylingmitteln sowie andere Verschmutzungen von der Haaroberfläche und der Kopfhaut.

Während der Reinigung werden aus dem Haar und der Kopfhaut aber auch Lipide und Proteine entfernt, wodurch insbesondere bei häufiger Reinigung eine Schädigung der Haarstruktur und ein Austrocknen der Kopfhaut erfolgen kann. Um diese Nachteile zu beseitigen wurden daher in den letzten Jahren bevorzugt solche Tenside in Haarreinigungsmitteln eingesetzt, die mild und auf dem Haar sowie der Kopfhaut gut verträglich sind.

Zwar sind an sich milde Tenside bekannt, doch befriedigen diese noch nicht alle Verbraucherwünsche in ausreichendem Maße. So hat sich beispielsweise gezeigt, dass einige Formulierungen auf der Basis milder Tenside sich nicht immer in der gewünschten Textur bereitstellen lassen. Um die gewünschte Textur zu erhalten, d.h. das gewünschte "cremige" Fließverhalten und eine höhere Viskosität der Mittel einzustellen, sind umfangreiche Formulierungsarbeiten vonnöten, und oft ist die Langzeitstabilität dieser Mittel nicht befriedigend. Des Weiteren sind milde Tenside oftmals nicht schaumstark und verringern die Schaummenge sowie die Schaumqualität (Cremigkeit und Feinporigkeit des Schaums). Die Einarbeitung von Pflegestoffen in Haarreinigungsmittel kann diesen Effekt noch verstärken.

Neben der Reinigung entscheidet vor allem die Wahrnehmung des Haares nach der Reinigung im nassen und trockenen Zustand über die Zufriedenheit eines Verbrauchers mit einem kosmetischen Haarreinigungsmittel. Entscheidend bei der Wahrnehmung ist das Gefühl der Pflege, das sich insbesondere durch den Griff, die Kämmbarkeit, den Glanz und die Geschmeidigkeit der Haare beschreiben lässt. Weiterhin sollen die Haare weder schwer erscheinen, noch elektrostatisch aufgeladen sein. Es besteht daher weiterhin ein Bedarf nach Wirkstoffen bzw. Wirkstoffkombinationen für kosmetische Reinigungszubereitungen mit guten pflegenden Eigenschaften, einem vorteilhaften Rheologieprofil und guten Schaumeigenschaften.

Kosmetische Zusammensetzungen, welche neben einem anionischen Tensid, einem amphoteren Tensid und einem Sorbitansärueester weiterhin kationisches Polymer enthalten, werden in den Patentanmeldungen WO 2010/108738 A2, EP 2 335 681 A1, EP 2 415 454 A1 und DE 10 2011 078 952 A1 beschrieben.

Der vorliegenden Anmeldung lag die Aufgabe zugrunde, milde, haut- und haarverträgliche und pflegende Haarreinigungsmittel mit verbesserter Textur und Schaumqualität bereitzustellen. Es wurde nun gefunden, dass diese Aufgabe durch eine spezifische Wirkstoffkombination aus anionischem Tensid Sorbitansesquioctanoat und kationischem Polysaccharid gelöst wird. Die entsprechenden Reinigungsmittel sind haut- und haarverträglich, pflegen und reinigen das Haar und lassen sich aufgrund ihrer vorteilhaften rheologischen Eigenschaften leicht auf dem Haar verteilen, und bilden in Verbindung mit Wasser einen cremigen, feinporigen Schaum.

Ein erster Gegenstand der Erfindung ist daher eine reinigende kosmetische Zusammensetzung, enthaltend in einem kosmetischen Träger bezogen auf sein Gesamtgewicht
a) mindestens ein anionisches Tensid (A),
b) gegebenenfalls amphoteres Tensid (B),
c) 0,25 bis 1,0 Gew.-% Sorbitansäureester (C), wobei die Sorbitansäureester Ester eines Sorbitans mit geradkettigen oder verzweigten, gesättigten oder ungesättigten Carbonsäuren mit 4 bis 10 Kohlenstoffatomen im Molekül, umfasst,
d) 0,1 bis 0,5 Gew.-% kationisches Polysaccharid (D), ), und
e) 0,01 bis 0,3 Gew.-% eines nativen Öls (E),
dadurch gekennzeichnet, dass der Gewichtsanteil des in der Zusammensetzung enthaltenen anionischen Tensids (A) und amphoteren Tensids (B), bezogen auf das Gesamtgewicht der Zusammensetzung 6,0 bis 11 Gew.-% beträgt.

Die erfindungsgemäßen reinigenden Kosmetika enthalten als ersten wesentlichen Bestandteil mindestens ein anionisches Tensid (A), wobei vorzugsweise nur vergleichsweise geringe Mengen eingesetzt werden. Der Gewichtsanteil des anionischen Tensids (A) und des gegebenenfalls vorhandenen amphoteren Tensids (B) am Gesamtgewicht der Zusammensetzung beträgt 6,0 bis 11,0 Gew.-%.

Zuden geeignetenanionischen Tensiden(A), die in den erfindungsgemäßen Mitteln eingesetztwerden können, zählen:
- lineare und verzweigte Fettsäuren mit 8 bis 30 C-Atomen (Seifen),
- Ethercarbonsäuren der Formel R-O-(CH₂-CH₂O)ₓ-CH₂-COOH, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel R-(OCH₂-CH₂)ₓ-OSO₃⁻ X⁺, in der R eine bevorzugt lineare oder verzweigte, gesättigte oder ungesättigte Alkylgruppe mit 8 bis 30 C-Atomen, x = 0 oder 1 bis 12 und X ein Alkali- oder Ammoniumion ist,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel, 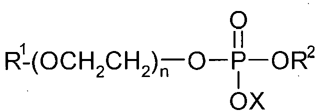 in der R¹ bevorzugt für einen aliphatischen Kohlenwasserstoffrest mit 8 bis 30 Kohlenstoffatomen, R² für Wasserstoff, einen Rest (CH₂CH₂O)ₙR¹ oder X, n für Zahlen von 0 bis 10 und X für Wasserstoff, ein Alkali- oder Erdalkalimetall oder NR³R⁴R⁵R⁶, mit R³ bis R⁶ unabhängig voneinander stehend für einen C₁ bis C₄ -Kohlenwasserstoffrest, steht.

Bevorzugte anionische Tenside sind Ethercarbonsäuren der zuvor genannten Formel, Acylsarcoside mit 8 bis 24 C-Atomen in der Acylgruppe, Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen, Alpha-Olefinsulfonate mit 8 bis 24 C-Atomen und/oder Alkylsulfat- und/oder Alkylpolyglykolethersulfatsalze der zuvor genannten Formel.

Besonders bevorzugte anionische Tenside sind geradkettige oder verzweigte Alkylethersulfate, die einen Alkylrest mit 8 bis 18 und insbesondere mit 10 bis 16 C-Atomen sowie 1 bis 6 und insbesondere 2 bis 4 Ethylenoxideinheiten enthalten. Weiterhin besonders bevorzugte anionische Tenside sind geradkettige oder verzweigte Alkylsulfonate, die einen Alkylrest mit 8 bis 18 und insbesondere mit 10 bis 16 C-Atomen enthalten. Insbesondere bevorzugt sind die Natrium-, Magnesium und/oder Triethanolaminsalze linearer oder verzweigter Lauryl-, Tridecyl- und/oder Myristylsulfate, die einen Ethoxylierungsgrad von 2 bis 4 aufweisen.

Ganz besonders bevorzugte erfindungsgemäße Kosmetika sind dadurch gekennzeichnet, dass sie mindestens ein anionisches Tensid (A) aus der Gruppe der Alkylsulfate und Alkylpolyglykolethersulfate der Formel R-O-(CH₂-CH₂O)n-O-SO₃X enthält, in der R bevorzugt für einen geradkettigen oder verzweigten, gesättigten oder ein- bzw. mehrfach ungesättigten Alkyl- oder Alkenylrest mit 8 bis 24 Kohlenstoffatomen, n für 0 oder 1 bis 12 und X für ein Alkali- oder Erdalkalimetall oder für Triethanolamin steht, wobei anionische Tenside mit der INCI Bezeichnung Sodium Lauryl Sulfate besonders bevorzugt sind.

Als zweiten wesentlichen Bestandteil enthalten die erfindungsgemäßen Kosmetika mindestens ein amphoteres Tensid (B). Amphotere Tenside werden den erfindungsgemäßen Mitteln - bezogen auf deren Gesamtgewicht - bevorzugt in Mengen von 1,0 oder 2,0 oder 3,0 Gew.-% zugegeben. Geeignete amphotere Tenside können ausgewählt sein aus Verbindungen der folgenden Formeln (i) bis (v), in denen der Rest R jeweils für einen geradkettigen oder verzweigten, gesättigten oder ein- bzw. mehrfach ungesättigten Alkyl- oder Alkenylrest mit 8 bis 24 Kohlenstoffatomen steht,

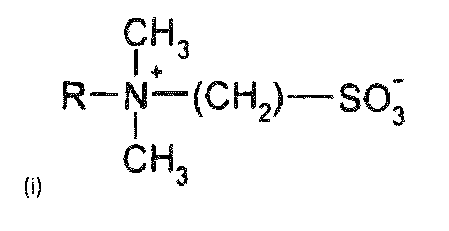

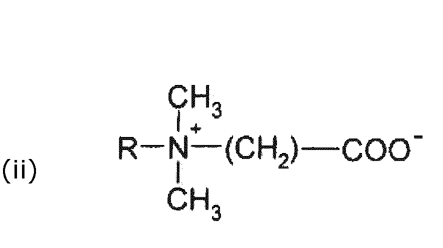

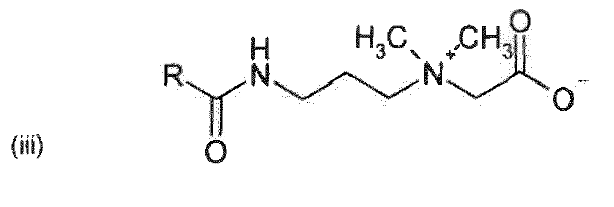

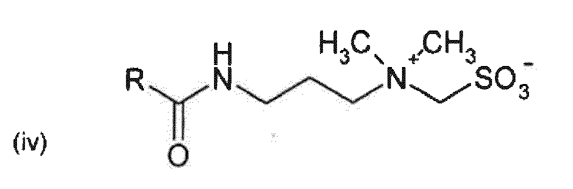

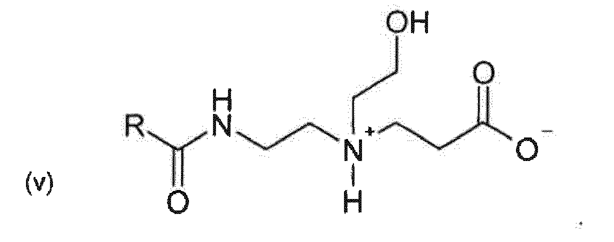

Besonders geeignete amphotere Tenside sind Alkylamidoalkylbetaine und/oder Alkylampho(di)acetate der zuvor genannten Formeln (i) bis (v). Zu den insbesondere geeigneten amphoteren Tensiden zählen die unter der INCI-Bezeichnung bekannten Tenside Cocamidopropylbetain und Disodium Cocoamphodiacetate. Besonders bevorzugte erfindungsgemäße Kosmetika sind dadurch gekennzeichnet, dass sie mindestens ein amphoteres Tensid (B) aus der Gruppe der allgemeinen Formel (iii) enthalten, wobei amphotere Tenside mit der INCI Bezeichnung Cocamidopropyl Betaine besonders bevorzugt sind.

Bevorzugte erfindungsgemäße Zusammensetzungen sind dadurch gekennzeichnet, dass der Gewichtsanteil des in der Zusammensetzung enthaltenen anionischen Tensids (A) und amphoteren Tensids (B), bezogen auf das Gesamtgewicht der Zusammensetzung 8,0 bis 11 Gew.-% beträgt.

Der dritte wesentliche Bestandteil der erfindungsgemäßen kosmetischen Zusammensetzungen mindestens einen Sorbitansäureester (C).

Die Sorbitansäureester (C), die in den erfindungsgemäßen Reinigungsmitteln eingesetzt werden, sind Ester des Sorbitans mit geradkettigen oder verzweigten, gesättigten oder ungesättigten Carbonsäuren mit 4 bis 10 Kohlenstoffatomen im Molekül.

Sorbitane leiten sich von dem Zuckeralkohol Sorbitol ab, der unter Abspaltung von Wasser cyclische innere Ether bildet. Sorbitane stellen üblicherweise ein Produktgemisch dar, das überwiegend aus Ethern der nachfolgenden Formeln (III) bis (V) besteht:

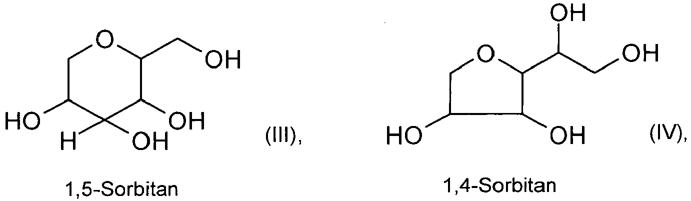

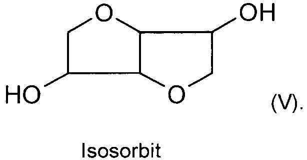

In untergeordneten Mengen enthalten Sorbitane auch weitere Kondensationsprodukte sowie Sorbitol.

Unter Sorbitanestern werden demnach bevorzugt Gemische der zuvor genannten Polyolgemische mit den zuvor genannten Carbonsäuren verstanden, wobei es weiterhin bevorzugt ist, wenn ein Mol des Polyolgemisches mit 1 bis 3 Mol Carbonsäure verestert wird.

Erfindungsgemäß besonders bevorzugte Zusammensetzungen enthalten zuvor beschriebene Sorbitanester (C), die durch Veresterung des Sorbitans mit Capronsäure, Önanthsäure, Caprylsäure, Pelargonsäure und/oder Caprinsäure gebildet werden. Besonders bevorzugt sind Sorbitanester, die aus Sorbitan und Caprylsäure gebildet werden.

Weiterhin besonders bevorzugte Reinigungsmittel enthalten zuvor beschriebene Sorbitancarbonsäureester, die bevorzugt eine Hydroxylzahl von mehr als 350, mehr bevorzugt von mehr als 400 und besonders bevorzugt von mehr als 450 aufweisen. Die Hydroxylzahl kann nach den üblichen und im Stand der Technik bekannten Bestimmungsverfahren ermittelt werden, beispielsweise nach DGF C-V 17a (53), Ph. Eur. 2.5.3 Method A und DIN 53240.

Besonders bevorzugte Zusammensetzungen enthalten mindestens einen Sorbitansäureester (C), mit der INCI-Bezeichnung Sorbitan Sesquicaprylat.

Ein letzter wesentlicher Bestandteil der erfindungsgemäßen kosmetischen Zusammensetzungen ist das kationische Polysaccharid (D).

Erfindungsgemäß bevorzugte kationische Polysaccharide (E) sind
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- hydrophob modifizierte Cellulosederivate, beispielsweise die unter dem Handelsnamen SoftCat^{®} vertriebenen kationischen Polymere,
- kationische Alkylpolyglycoside,
- kationisierter Honig, beispielsweise das Handelsprodukt Honeyquat^{®} 50,
- kationische Guar-Derivate, wie insbesondere die unter den Handelsnamen Cosmedia^{®}Guar und Jaguar^{®} vertriebenen Produkte,

In einer bevorzugten Ausführungsform der Erfindung enthalten die Zusammensetzungen weiterhin mindestens ein zuvor genanntes kationisches quaternisiertes Cellulosepolymer und/oder ein kationisches Guarderivat. Bevorzugt sind die quaternisierten Cellulosepolymere, die unter der INCI-Bezeichnung Polyquaternium-10 bekannt sind (Polymer JR^{®}- oder Celquat^{®}-Polymere).

Besonders bevorzugte kationische Polysaccharide (E) sind die kationischen Hydroxyalkyl-Guar-Derivate, vorzugsweise kationisches Hydroxyethyltrimethylammonium-Guar und/oder kationisches Hydroxypropyltrimethylammonium-Guar mit mittleren Molekulargewichten zwischen 800.000 und 1.600.000 Dalton. Insbesondere bevorzugt sind die unter der INCI-Bezeichnung Guar Hydroxypropyltrimonium Chlorid bekannten kationischen Guar-Polymere mit einem Molekulargewicht (Gewichtsmittel) zwischen 800.000 und 1.600.000 Dalton. Die kationische Ladungsdichte dieser Guar-Polymere beträgt vorzugsweise mindestens 0,5 meq/g, bevorzugt mindestens 0,6 meq/g und insbesondere mindestens 0,8 meq/g. Ihr Stickstoffgehalt liegt vorzugsweise im Bereich von 1,1 bis 1,6 Gew.-% (bezogen auf ihr Gesamtgewicht). Zusammenfassend werden erfindungsgemäße kosmetische Zusammensetzungen bevorzugt, die als kationisches Polysaccharid (E) ein kationisches Guar-Derivat enthalten.

(gestrichen)

Die erfindungsgemäßen Zusammensetzungen enthalten die Komponenten a) bis f) in einem kosmetischen Träger,derbevorzugtwässrigoderwässrig-alkoholischseinkann. BevorzugtenthältderkosmetischeTräger mindestens 40 Gew.-%, vorzugsweise 60 bis 95 Gew.-% und insbesondere 75 bis 90 Gew.-% Wasser.

Weiterhin kann der kosmetische Träger mindestens einen Alkohol enthalten, der ausgewählt sein kann aus Ethanol, Ethyldiglykol, 1-Propanol, 2-Propanol, Isopropanol, 1,2-Propylenglycol, Glycerin, 1-Butanol, 2-Butanol, 1,2-Butandiol, 1,3-Butandiol, 1-Pentanol,2-Pentanol, 1,2-Pentandiol, 1,5-Pentandiol, 1, Hexanol,2-Hexanol, 1,2-Hexandiol, 1,6-Hexandiol, Sorbitol, Benzylalkohol, Phenoxyethanol oder Mischungen dieser Alkohole. Bevorzugt sind die wasser-löslichen Alkohole. Insbesondere bevorzugt sind Ethanol, Ethyldiglykol, 1-Propanol, 2-Propanol, Isopropanol, 1,2-Pro-pylenglycol, Glycerin, Benzylalkohol und/oder Phenoxyethanol sowie Mischungen dieser Alkohole.

Neben den zuvor genannten Wirk- und Trägerstoffen können die erfindungsgemäßen kosmetischen Zusammensetzungen noch eine Reihe weiterer Inhaltsstoffe enthalten, die ihnen vorteilhafte Eigenschaften verleihen. Zu den bevorzugten fakultativen Wirkstoffen, die in den erfindungsgemäßen Zusammensetzungen eingesetzt werden können, zählen beispielsweise:
- nichtionische Tenside und/oder nichtionische Emulgatoren, die in den kosmetischen Zusammensetzungen (bezogen auf ihr Gesamtgewicht) bevorzugt in einer Menge von 0,1 bis 7,5 Gew.-%, bevorzugt 0,25 bis 5 Gew.-% und insbesondere 0,5 bis 3 Gew.-% eingesetzt werden können,
- weitere Öl-, Wachs- und/oder Fettkomponenten, die in den kosmetischen Zusammensetzungen (bezogen auf ihr Gesamtgewicht) bevorzugt in einer Menge von 0,01 - 20 Gew.-%, besonders bevorzugt von 0,05 - 15 Gew.% und insbesondere von 0,1 - 10 Gew.% eingesetzt werden können,
- Antischuppenwirkstoffe, die in den in den kosmetischen Zusammensetzungen (bezogen auf ihr Gesamtgewicht) bevorzugt in einer Menge von 0,025 bis 7,5 Gew.-%, besonders bevorzugt von 0,05 bis 5 Gew.-% und insbesondere von 0,075 bis 3 Gew.-% eingesetzt werden können.

Zu den geeigneten nichtionischen Tensiden/Emulgatoren zählen beispielsweise
- C₈-C₃₀-Fettsäuremono- und -diester von Anlagerungsprodukten von 1 bis 30 Mol Ethylenoxid an Glycerin,
- Aminoxide,
- Anlagerungsprodukte von 2 bis 50 Mol Ethylenoxid und/oder 0 bis 5 Mol Propylenoxid an lineare und verzweigte Fettalkohole mit 8 bis 30 C-Atomen, an Fettsäuren mit 8 bis 30 C-Atomen und an Alkylphenole mit 8 bis 15 C-Atomen in der Alkylgruppe,
- Sorbitanfettsäureester und Anlagerungeprodukte von Ethylenoxid an Sorbitanfettsäureester wie beispielsweise Polysorbate,
- Zuckerfettsäureester und Anlagerungsprodukte von Ethylenoxid an Zuckerfettsäureester,
- Fettsäurealkanolamide,
- Anlagerungsprodukte von Ethylenoxid an Fettamine und/oder
- Alkylpolyglucoside.

Besonders geeignete nichtionische Tenside sind Alkyloligoglucoside, insbesondere Alkyloligoglucoside auf der Basis von gehärtetem C_{12/14}-Kokosalkohol mit einem DP von 1-3, wie sie beispielsweise unter der INCI-Bezeichnung "Coco-Glucoside" im Handel erhältlich sind, und Fettsäurealkanolamide, insbesondere C₈-C₂₄-Fettsäure(di)ethanolamide.

Weiterhin bevorzugte nichtionische Tenside sind die C₈-C₃₀-Fettsäuremono- und -diester von Anlagerungsprodukten von 1 bis 30 Mol Ethylenoxid an Glycerin. Besonders bevorzugt sind die C₁₀-C₁₆-Fettsäuremono- und -diester von Anlagerungsprodukten von 1 bis 10 Mol Ethylenoxid an Glycerin. Insbesondere bevorzugt ist das unter der INCI-Bezeichnung bekannte PEG-7 Glyceryl Cocoate. Bevorzugte Zusammensetzungen sind dadurch gekennzeichnet, dass sie zusätzlich ein ethoxyliertes nichtionisches Tensid mit einem HLB-Wert von 12 bis 18 enthalten.

Geeignete Öl- und/oder Fettkomponenten können bevorzugt ausgewählt sein aus mineralischen, natürlichen und synthetischen Ölkomponenten und/oder Fettstoffen.

Als natürliche (pflanzliche) Öle werden üblicherweise Triglyceride und Mischungen von Triglyceriden eingesetzt. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaobutter und Shea-Butter. Die erfindungsgemäßen Zusammensetzungen sind dadurch gekennzeichnet, dass sie weiterhin ein natives Öl enthalten, wobei der Gewichtsanteil des Öls am Gesamtgewicht der Zusammensetzung 0,01 bis 0,3 Gew.-%, vorzugsweise von 0,05 bis 0,1 Gew.-% beträgt. Ein besonders bevorzugtes natives Öl ist Jojobaöl.

Als mineralische Öle kommen insbesondere Mineralöle, Paraffin- und Isoparaffinöle sowie synthetische Koh-Ienwasserstoffe zum Einsatz. Ein Beispiel für einen einsetzbaren Kohlenwasserstoff ist beispielsweise das als Handels-produkt erhältliche 1,3-Di-(2-ethylhexyl)-cyclohexan (Cetiol^{®} S).

Als Ölkomponente kann weiterhin ein Dialkylether dienen. Einsetzbare Dialkylether sind insbesondere Din-alkylether mit insgesamt zwischen 12 bis 36 C-Atomen, insbesondere 12 bis 24 C-Atomen, wie beispielsweise Di-n-octylether, Di-n-decylether, Di-n-nonylether, Di-n-undecylether, Di-n-dodecylether, n-Hexyl-n-octylether, n-Octyl-n-de-cylether, n-Decyl-n-undecylether, n-Undecyl-n-dodecylether und n-Hexyl-n-undecylether sowie Di-tert.-butylether, Di-iso-pentylether, Di-3-ethyldecylether, tert.-Butyl-n-octylether, iso-Pentyl-n-octylether und 2-Methylpentyl-n-octylether. Besonders bevorzugt ist der Di-n-octylether, der im Handel unter der Bezeichnung Cetiol^{®} OE erhältlich ist.

Als synthetische Öle kommen Silikonverbindungen in Betracht.

Silikone bewirken auf dem Haar ausgezeichnete konditionierende Eigenschaften. Insbesondere bewirken sie eine bessere Kämmbarkeit der Haare in nassem und trockenem Zustand und wirken sich in vielen Fällen positiv auf den Haargriff und die Weichheit der Haare aus.

Es ist daher erstrebenswert, in den kosmetischen Zusammensetzungen Silikone einzusetzen. Geeignete Silikone (die sich von den Silikonen a) unterscheiden) können ausgewählt sein unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Cärboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopolymeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Als synthetisches Öl eignen sich insbesondere die Polydimethylsiloxane. Zur Gruppe der Polydimethylsiloxane zählen die linearen wie die nichtlinearen, vorzugsweise cyclischen Polydimethylsiloxane. Erfindungsgemäß bevorzugte Polydimethylsiloxane sind ausgewählt aus den linearen Polydimethylsiloxanen mit einer kinematischen Viskosität (25°C) oberhalb 8000 cst, vorzugsweise oberhalb 10000 cst. Das Polydimethylsiloxan liegt in der kosmetischen Zusammensetzung vorzugsweise in dispergierter Form vor, wobei die Tröpfchengröße des dispergierten Polydimethylsiloxans weniger als 10 µm, vorzugsweise weniger als 4 µm beträgt.

Unter Fettstoffen sind zu verstehen Fettsäuren, Fettalkohole sowie natürliche und synthetische Wachse, welche sowohl in fester Form als auch flüssig in wässriger Dispersion vorliegen können.

Als Fettsäuren können eingesetzt werden lineare und/oder verzweigte, gesättigte und/oder ungesättigte Fettsäuren mit 6 - 30 Kohlenstoffatomen. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.

Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Als Fettalkohole können eingesetzt werden gesättigte, ein- oder mehrfach ungesättigte, verzweigte oder unverzweigte Fettalkohole mit C₆ - C₃₀-, bevorzugt C₁₀ - C₂₂- und ganz besonders bevorzugt C₁₂ - C₂₂- Kohlenstoffatomen. Einsetzbar sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll.

Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Als natürliche oder synthetische Wachse können eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

### Weitere Fettstoffe sind beispielsweise

- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen.
   Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen. Besonders bevorzugt sind Isopropylmyristat (Rilanit^{®} IPM), Isononansäure-C16-18-alkylester (Cetiol^{®} SN), 2-Ethylhexylpalmitat (Cegesoft^{®}24), Stearinsäure-2-ethylhexylester (Cetiol^{®} 868), Cetyloleat, Glycerintricaprylat, Kokosfettalkohol- caprinat/-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen,
- Glycerincarbonat oder Dicaprylylcarbonat (Cetiol^{®} CC),
- ethoxylierte oder nicht ethoxylierte Mono,- Di- und Trifettsäureester von gesättigten und/oder ungesättigten linearen und/oder verzweigten Fettsäuren mit Glycerin, wie beispielsweise Monomuls^{®} 90-018, Monomuls^{®} 90-L12, Cetiol^{®} HE oder Cutina^{®} MD.

Geeignete Antischuppenwirkstoffe können ausgewählt sein aus Piroctone Olamine, Climbazol, Zink Pyrithion, Ketoconazole, Salicylsäure, Schwefel, Selensulfid, Teerpräparaten, Undecensäurederivaten, Klettenwurzelextrakten, Pappelextrakten, Brennesselextrakten, Walnussschalenextrakten, Birkenextrakten, Weidenrindenextrakten, Rosmarinextrakten und/oder Arnikaextrakten. Bevorzugt sind Climbazol, Zink Pyrithion und Piroctone Olamine.

Zu den weiteren fakultativen Komponenten, die in den erfindungsgemäßen Zusammensetzungen eingesetzt werden können, zählen beispielsweise
- Vitamine, Vitaminderivate und/oder Vitaminvorstufen,
- Pflanzenextrakte und/oder
- Feuchthaltemittel.

Unter geeigneten Vitaminen sind bevorzugt die folgenden Vitamine, Provitamine und Vitaminvorstufen sowie deren Derivate zu verstehen:
Vitamin A: zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente kommen beispielsweise Vitamin A-Säure und deren Ester, Vitamin A-Aldehyd und Vitamin A-Alkohol sowie dessen Ester wie das Palmitat und das Acetat in Betracht.
Vitamin B: zur Vitamin B-Gruppe oder zu dem Vitamin B-Komplex gehören u. a.
   - Vitamin B₁ (Thiamin)
   - Vitamin B₂ (Riboflavin)
   - Vitamin B₃. Unter dieser Bezeichnung werden häufig die Verbindungen Nicotinsäure und Nicotinsäureamid (Niacinamid) geführt.
   - Vitamin B₅ (Pantothensäure und Panthenol). Im Rahmen dieser Gruppe wird bevorzugt das Panthenol eingesetzt. Einsetzbare Derivate des Panthenols sind insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate.
   - Vitamin B₆ (Pyridoxin sowie Pyridoxamin und Pyridoxal).
Vitamin C (Ascorbinsäure): die Verwendung in Form des Palmitinsäureesters, der Glucoside oder Phosphate kann bevorzugt sein. Die Verwendung in Kombination mit Tocopherolen kann ebenfalls bevorzugt sein.
Vitamin E (Tocopherole, insbesondere α-Tocopherol).
Vitamin F: unter dem Begriff "Vitamin F" werden üblicherweise essentielle Fettsäuren, insbesondere Linolsäure, Linolensäure und Arachidonsäure, verstanden.
Vitamin H: Als Vitamin H wird die Verbindung (3aS,4S,6aR)-2-Oxohexahydrothienol[3,4-d]-imidazol-4-valeriansäure bezeichnet, für die sich aber zwischenzeitlich der Trivialname Biotin durchgesetzt hat. Die erfindungsgemäßen kosmetischen Zusammensetzungen können bevorzugt Vitamine, Provitamine und Vitaminvorstufen aus den Gruppen A, B, E und H enthalten.

Insbesondere bevorzugt sind Nicotinsäureamid, Biotin, Pantolacton und/oder Panthenol.

Vitamine, Vitaminderivate und/oder Vitaminvorstufen können in den Zusammensetzungen (bezogen auf ihr Gesamtgewicht) bevorzugt in einer Menge von 0,001 bis 10 Gew.-%, bevorzugt 0,005 bis 7,5 Gew.-% und insbesondere 0,01 bis 5 Gew.-% eingesetzt werden.

Unter geeigneten Pflanzenextrakten sind Extrakte zu verstehen, die aus allen Teilen einer Pflanze hergestellt werden können. Üblicherweise werden diese Extrakte durch Extraktion der gesamten Pflanze hergestellt. Es kann aber in einzelnen Fällen auch bevorzugt sein, die Extrakte ausschließlich aus Blüten und/oder Blättern der Pflanze herzustellen. Geeignet sind vor allem die Extrakte aus Grünem Tee, Eichenrinde, Brennessel, Hamamelis, Hopfen, Kamille, Klettenwurzel, Schachtelhalm, Weißdorn, Lindenblüten, Litschi, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Ginseng, Ingwerwurzel, Echinacea purpurea, Olea europea, Boerhavia Diffusa-Wurzeln, Foeniculum vulgaris und Apim graveolens.

Besonders bevorzugt für die Verwendung in den erfindungsgemäßen Zusammensetzungen sind die Extrakte aus Grünem Tee, Brennessel, Hamamelis, Kamille, Aloe Vera, Ginseng, Echinacea purpurea, Olea europea und/oder Boerhavia Diffusa-Wurzeln.

Als Extraktionsmittel zur Herstellung der genannten Pflanzenextrakte können Wasser, Alkohole sowie deren Mischungen verwendet werden. Unter den Alkoholen sind dabei niedere Alkohole wie Ethanol und Isopropanol, insbesondere aber mehrwertige Alkohole wie Ethylenglykol und Propylenglykol, sowohl als alleiniges Extraktionsmittel als auch in Mischung mit Wasser, bevorzugt. Pflanzenextrakte auf Basis von Wasser/Propylenglykol im Verhältnis 1:10 bis 10:1 haben sich als besonders geeignet erwiesen.

Die Pflanzenextrakte können sowohl in reiner als auch in verdünnter Form eingesetzt werden. Sofern sie in verdünnter Form eingesetzt werden, enthalten sie üblicherweise ca. 2 - 80 Gew.-% Aktivsubstanz und als Lösungsmittel das bei ihrer Gewinnung eingesetzte Extraktionsmittel oder Extraktionsmittelgemisch.

Die Pflanzenextrakte können in den erfindungsgemäßen Zusammensetzungen (bezogen auf ihr Gesamtgewicht) bevorzugt in einer Menge von 0,01 bis 10 Gew.-%, mehr bevorzugt von 0,05 bis 7,5 Gew.-% und insbesondere von 0,1 bis 5 Gew.-% eingesetzt werden.

Geeignete Feuchthaltemittel bzw. Penetrationshilfsstoffe und/ oder Quellmittel, die den erfindungsgemäßen Reinigungsmitteln zugesetzt werden können, sind beispielsweise Harnstoff und Harnstoffderivate, Guanidin und dessen Derivate, Arginin und dessen Derivate, Wasserglas, Imidazol und Dessen Derivate, Histidin und dessen Derivate, Benzylalkohol, Glycerin, Glykol und Glykolether, Propylenglykol und Propylenglykolether, beispielsweise Propylenglykolmonoethylether, Carbonate, Hydrogencarbonate, Diole und Triole, und insbesondere 1,2-Diole und 1,3-Diole wie beispielsweise 1,2-Propandiol, 1,2-Pentandiol, 1,2-Hexandiol, 1,2-Dodecandiol, 1,3-Propandiol, 1,6-Hexandiol, 1,5-Pentandiol, 1,4-Butandiol.

Die Feuchthaltemittel können in den erfindungsgemäßen Zusammensetzungen - bezogen auf deren Gesamtgewicht - bevorzugt in Mengen von 0,01 bis 10 Gew.-%, mehr bevorzugt in Mengen von 0,05 bis 5 Gew.-% und insbesondere in Mengen von 0,1 bis 3 Gew.-% eingesetzt werden.

Weitere Wirk-, Hilfs- und Zusatzstoffe, die in den erfindungsgemäßen Haarreinigungsmitteln eingesetzt werden können, sind beispielsweise:
- UV-Filter,
- Verdickungsmittel wie Gelatine oder Pflanzengumme, beispielsweise Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone und Schichtsilikate wie z. B. Bentonit oder vollsynthetische Hydrokolloide wie z. B. Polyvinylalkohol, die Ca-, Mg- oder Zn - Seifen,
- Strukturanten wie Maleinsäure und Milchsäure,
- Farbstoffe zum Anfärben des Mittels,
- Substanzen zur Einstellung des pH-Wertes, beispielsweise α- und β-Hydroxycarbonsäuren wie Citronensäure, Milchsäure, Äpfelsäure, Glycolsäure,
- Wirkstoffe wie Bisabolol,
- Komplexbildner wie EDTA, NTA, β-Alanindiessigsäure und Phosphonsäuren,
- Ceramide. Unter Ceramiden werden N-Acylsphingosin (Fettsäureamide des Sphingosins) oder synthetische Analogen solcher Lipide (sogenannte Pseudo-Ceramide) verstanden,
- Treibmittel wie Propan-Butan-Gemische, N₂O, Dimethylether, CO₂ und Luft,
- Antioxidantien,
- Perlglanzmittel wie EGDS oder PEG-3 Distearate,
- Konservierungsmittel, wie beispielsweise Natriumbenzoat oder Salicylsäure,
- Viskositätsregler wie Salze (NaCl).

Die erfindungsgemäßen kosmetischen Zusammensetzungen weisen bevorzugt pH-Werte im Bereich von 2 bis 7, bevorzugt von 3 bis 6 und insbesondere von 4,5 bis 5,5 auf.

(gestrichen)

Wie eingangs ausgeführt, weisen die erfindungsgemäßen Reinigungsmittel hervorragende Eigenschaften in der Anwendung auf den Haaren auf. Neben der Reinigung verleihen sie den damit behandelten Haaren bessere optische und haptische Eigenschaften. Behandelte trockene Haare (insbesondere geschädigte Haare) weisen einen verbesserten Haargriff und eine verbesserte Kämmbarkeit auf. Behandelte nasse Haare (insbesondere geschädigte Haare) weisen weniger bzw. keine Verknotungen auf und lassen sich leicht entwirren. Ein weiterer Vorteil der erfindungsgemäßen Reinigungsmittel ist, dass sie die Widerstandfähigkeit der Haaroberfläche gegen physikalische und/oder chemische Haarschädigungen unterstützen.

Ein weiterer Gegenstand der vorliegenden Anmeldung ist ein Verfahren zur Haarbehandlung, dadurch gekennzeichnet, dass eine erfindungsgemäße Reinigungszusammensetzung auf die nassen Haareaufgebrachtund nach einer Einwirkungszeit von 30 Sekunden bis 5 Minuten mit Wasser ausgespült wird.

Ein weiterer Gegenstand ist die Verwendung einer erfindungsgemäßen kosmetischen Zusammensetzung zur Verbesserung des Griffs und der Kämmbarkeit nasser und trockener Haare sowie zur Verbesserung der Widerstandskraft der Haaroberfläche gegen physikalische und/ oder chemische Haarschädigungen
Beispiele:

### 1) Herstellung eines erfindungsgemäßen Shampoos:

| | I |
|---|---|
| Sodium Laureth Sulfate | 10,8 |
| Sorbitan Sesquicaprylate | 0,9 |
| Coconut glycerides +7.3 EO | 0,3 |
| PEG-12 Dimethicone | 0,5 |
| Polyquaternium-10 | 0,7 |
| PEG-40 Hydrogenated Castor Oil | 0,4 |
| Aprikosenkernöl | 0,02 |
| Parfum, Konservierungsmittel | q.s. |
| Citronensäure (bis pH 4,7 bis 5,3) | 0,2 |
| NaCl (bis Viskosität 5,000 bis 8,000 [Brookfield 20 RM/20°C] +/- 1000 mPas) | 3,9 |
| Wasser | ad 100 |

### 2) Beurteilung der Shampoos:

### 2.1 Beurteilung der Nasskämmbarkeit

a) Haarsträhnen / Messgerät: Es wurden Haarsträhnen der Firma Kerling International (Backnang, Deutschland) verwendet: European natural hair 7/0; lot #04/2010; N74; Länge: 12 cm; Gewicht: 1 +/-0,05g. Messgerät: Hercules Sägemann (Hamburg, Deutschland) und Hartgummikamm, feinzahnig
b) Behandlung der Haarsträhnen und Messung: Die Haarsträhnen wurden zunächst vorgeschädigt, indem sie zweimal gebleicht und anschließend bei 25°C und 25% Luftfeuchtigkeit für 48 Stunden aufbewahrt wurden. 0,25 g des jeweiligen Shampoos wurde pro g Haarsträhne aufgebracht. Nach 2 Minuten wurden die Haarsträhnen mit Wasser (32°C, 0,5l/min) für 18 Sekunden gespült, während sie automatisch gekämmt wurden. Diese Prozedur wurde noch einmal wiederholt. Vor der Messung wurde jede Haarsträhne für 2 Sekunden mit Wasser befeuchtet. Nachdem jede Haarsträhne vorgekämmt wurde (3 Striche), wurde jede Haarsträhne 10 Mal gekämmt, wobei die dabei aufgewendete Arbeit gemessen wurde (dabei wurden die Haarsträhnen langsam gedreht). Die aufgewendete Arbeit wurde in Bezug zur Verringerung der Nasskämmbarkeit gesetzt. (dabei gilt: je höher der Zahlenwert für die Verringerung der Nasskämmbarkeit ist, desto niedriger war die aufzuwendende Arbeit, um den Kamm durch die Haarsträhne zu ziehen). Für jedes Shampoo wurde die Nasskämmbarkeit an 12 Haarsträhnen gemessen.

### 2.2 Beurteilung der Entwirrung und des Haargriffs in Haarsträhnentests durch Experten

In einem zweiten Test wurden in acht Versuchsreihen jeweils drei Haarsträhnen von zwei unabhängigen Experten (a,b) in Bezug auf die Entwirrbarkeit (in nassem Zustand) und den Griff (Weichheit, Geschmeidigkeit) der Haarsträhnen in trockenem Zustand beurteilt. Bei den verwendeten Haarsträhnen handelte es sich um Maschinentressen aus Euro-Natur-Haar; remis; doppelt gezogen; Farbe 7/0; 3 cm Breite, 25 cm Gesamtlänge; ca. 2 g Gesamtgewicht (Fa. Kerling Int. Haarfabrik GmbH). Die Haarsträhnen wurden vorgeschädigt, indem sie mit einem Haarfärbemittel (80 g "Igora Royal color & care developer", der mit 20 g "Igora blue dust-free bleach" gemischt wurde) behandelt, und nach 30 Minuten für 20 Sekunden mit Wasser gespült wurden. Anschließend wurden die Strähnen jeweils mit 1ml einer 10%igen Natriumlaurylethersulfatlösung gewaschen, für 20 Sekunden mit Wasser gespült und ausgewrungen. 1g der jeweiligen Testshampoos wurde in jeweils eine Petrischale gefüllt. Anschließend wurden die vorgeschädigten Haarsträhnen kurz in die jeweiligen Testshampoos getaucht und mit Wasser ausgespült. Die Strähnen wurden von den beiden Experten in trockenem und in nassem Zustand beurteilt.

Die mit der erfindungsgemäßen Zusammensetzung behandelten Haarsträhnen zeichneten sich durch eine unerwartet gute Nasskämmbarkeit, gute Entwirrbarkeit und große Geschmeidigkeit aus.

## Patentansprüche

1. Reinigende kosmetische Zusammensetzung, enthaltend in einem kosmetischen Träger bezogen auf sein Gesamtgewicht
a) mindestens ein anionisches Tensid (A),
b) gegebenenfalls amphoteres Tensid (B),
c) 0,25 bis 1,0 Gew.-% Sorbitansäureester (C), wobei die Sorbitansäureester Ester eines Sorbitans mit geradkettigen oder verzweigten, gesättigten oder ungesättigten Carbonsäuren mit 4 bis 10 Kohlenstoffatomen im Molekül, umfasst,
d) 0,1 bis 0,5 Gew.-% kationisches Polysaccharid (D), und
e) 0,01 bis 0,3 Gew.-% eines nativen Öls (E),
**dadurch gekennzeichnet, dass** der Gewichtsanteil des in der Zusammensetzung enthaltenen anionischen Tensids (A) und amphoteren Tensids (B), bezogen auf das Gesamtgewicht der Zusammensetzung 6,0 bis 11 Gew.-% beträgt.

2. Zusammensetzung nach Anspruch 1, **dadurch gekennzeichnet, dass** sie mindestens ein anionisches Tensid (A) aus der Gruppe der Alkylsulfate und Alkylpolyglykolethersulfate der Formel R-O-(CH₂-CH₂-O)ₙ-O-SO₃X enthält, in der R bevorzugt für einen geradkettigen oder verzweigten, gesättigten oder ein- bzw. mehrfach ungesättigten Alkyl-oder Alkenylrest mit 8 bis 24 Kohlenstoffatomen, n für 0 oder 1 bis 12 und X für ein Alkali- oder Erdalkalimetall oder für Triethanolamin steht, wobei anionische Tenside mit der INCI Bezeichnung Sodium Lauryl Sulfate besonders bevorzugt sind.

3. Zusammensetzung nach einem der vorherigen Ansprühe, **dadurch gekennzeichnet, dass** sie mindestens ein amphoteres Tensid (B) aus der Gruppe der allgemeinen Formel (iii) 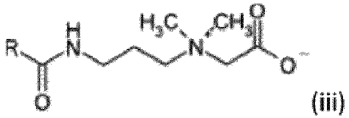 enthält, wobei amphotere Tenside mit der INCI Bezeichnung Cocamidopropyl Betaine besonders bevorzugt sind.

4. Zusammensetzung nach einem der vorherigen Ansprüche, **dadurch gekennzeichnet, dass** der Gewichtsanteil des in der Zusammensetzung enthaltenen an ionischen Tensids (A) und amphoteren Tensids (B), bezogen auf das Gesamtgewicht der Zusammensetzung 8,0 bis 11 Gew.-% beträgt.

5. Zusammensetzung nach einem der vorherigen Ansprüche, **dadurch gekennzeichnet, dass** als Sorbitansäureester (C) Sorbitansesquioctanoat eingesetzt wird.

6. Zusammensetzung nach einem der vorherigen Ansprüche, **dadurch gekennzeichnet, dass** sie mindestens ein kationisches Polysaccharid (D) aus der Gruppe der kationischen Guar-Derivate enthält.

7. Zusammensetzung nach einem der vorherigen Ansprüche, **dadurch gekennzeichnet, dass** sie weiterhin ein natives Öl (E) enthält, wobei der Gewichtsanteil des Öls (E) am Gesamtgewicht der Zusammensetzung vorzugsweise von 0,05 bis 0,1 Gew.-% beträgt.

8. Verfahren zur Haarbehandlung, **dadurch gekennzeichnet, dass** eine kosmetische Reinigungszusammensetzung nach einem der Ansprüche 1 bis 7 auf die nassen Haare aufgebracht, und nach einer Einwirkungszeit von 30 Sekunden bis 5 Minuten mit Wasser ausgespült wird.

## Claims

1. A cleaning cosmetic composition containing, in a cosmetic carrier and based on its total weight:
a) at least one anionic surfactant (A),
b) optionally an amphoteric surfactant (B),
c) 0.25 to 1.0 wt.% sorbitan acid ester (C), wherein the sorbitan acid ester comprises esters of a sorbitan with straight or branched, saturated or unsaturated carboxylic acids having 4 to 10 carbon atoms in its molecule,
d) 0.1 to 0.5 wt.% cationic polysaccharide (D) and
e) 0.01 to 0.3 wt.% a native oil (E),
**characterized in that** the proportion by weight of the anionic surfactant (A) and amphoteric surfactant (B) contained in the composition, based on the total weight of the composition, is 6.0 to 11 wt.%.

2. The composition according to claim 1, **characterized in that** it contains at least one at least one anionic surfactant (A) from the group of alkyl sulfates and alkyl polyglycol ether sulfates of formula R-O-(CH₂-CH₂O)ₙ-O-SO₃X, in which R is preferably a straight-chain or branched, saturated or mono- or polyunsaturated alkyl or alkenyl functional group having 8 to 24 carbon atoms, n is 0 or 1 to 12 and X is an alkali metal or alkaline earth metal or triethanolamine, anionic surfactants having the INCI designation sodium lauryl sulfate being particularly preferred.

3. The composition according to one of the preceding claims, **characterized in that** it contains at least one amphoteric surfactant (B) from the group of general formula (iii), 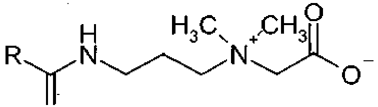 amphoteric surfactants having the INCI designation cocamidopropyl betaine being particularly preferred.

4. The composition according to one of the preceding claims, **characterized in that** the proportion by weight of the anionic surfactant (A) and amphoteric surfactant (B) contained in the composition, based on the total weight of the composition, is 8.0 to 11 wt.%.

5. The composition according to one of the preceding claims, **characterized in that** sorbitan sesqui octanoate is used as the sorbitan acid ester (C).

6. The composition according to one of the preceding claims, **characterized in that** it contains at least one cationic polysaccharide (D) from the group of cationic guar derivatives.

7. The composition according to one of the preceding claims, **characterized in that** it additionally contains a native oil (E), the proportion by weight of the oil (E) in the total weight of the composition being preferably 0.05 to 0.1 wt.%.

8. A method for hair treatment, **characterized in that** a cosmetic cleaning composition according to one of claims 1 to 7 is applied to wet hair, and is rinsed out using water after a contact time of from 30 seconds to 5 minutes.

## Revendications

1. Composition cosmétique nettoyante, contenant dans un support cosmétique sur la base de son poids total
a) au moins un tensioactif anionique (A),
b) éventuellement un tensioactif amphotère (B),
c) 0,25 à 1,0 % en poids d'ester d'acide de sorbitane (C), dans laquelle l'ester d'acide de sorbitane comprend des esters d'un sorbitan avec des acides carboxyliques à chaîne droite ou ramifiée, saturés ou insaturés ayant 4 à 10 atomes de carbone dans la molécule,
d) 0,1 à 0,5 % en poids de polysaccharide cationique (D) et
e) 0,01 à 0,3 % en poids d'huile native (E),
**caractérisée en ce que** la proportion en poids du tensioactif anionique (A) et du tensioactif amphotère (B) contenus dans la composition est de 6,0 à 11 % en poids, sur la base du poids total de la composition.

2. Composition selon la revendication 1, **caractérisée en ce qu'**elle contient au moins un tensioactif anionique (A) choisi dans le groupe constitué par des alkylsulfates et des alkylpolyglycoléthersulfates de formule R-O-(CH₂-CH₂O)ₙ-O-SO₃X, dans laquelle R est de préférence linéaire ou ramifié, saturé ou mono- ou polyinsaturé ayant 8 à 24 atomes de carbone, n vaut 0 ou 1 à 12 et X est un métal alcalin ou alcalino-terreux ou triéthanolamine, les tensioactifs anioniques ayant le nom INCI Sodium Lauryl Sulfate étant particulièrement préférés.

3. Composition selon l'une des revendications précédentes, **caractérisée en ce qu'**elle contient au moins un tensioactif amphotère (B) choisi dans le groupe de la formule générale (iii) 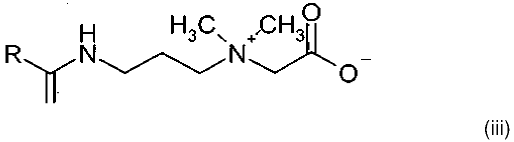 les tensioactifs amphotères ayant le nom INCI Cocamidopropyl Betaine étant particulièrement préférés.

4. Composition selon l'une des revendications précédentes, **caractérisée en ce que** la proportion en poids du tensioactif anionique (A) et du tensioactif amphotère (B) contenus dans la composition est de 8,0 à 11 % en poids, sur la base du poids total de la composition.

5. Composition selon l'une quelconque des revendications précédentes, **caractérisée en ce que** l'ester d'acide de sorbitane (C) utilisé est le sesquioctanoate de sorbitane.

6. Composition selon l'une des revendications précédentes, **caractérisée en ce qu'**elle contient au moins un polysaccharide cationique (D) choisi dans le groupe constitué par des dérivés de guar cationiques.

7. Composition selon l'une des revendications précédentes, **caractérisée en ce qu'**elle contient également une huile native (E), la proportion en poids de l'huile (E) par rapport au poids total de la composition étant de préférence de 0,05 à 0,1 % en poids.

8. Procédé de traitement capillaire, **caractérisé en ce qu'**une composition nettoyante cosmétique est appliquée sur les cheveux mouillés selon l'une des revendications 1 à 7, et rincée après un temps de contact de 30 secondes à 5 minutes avec de l'eau.
